# EUROPEAN PATENT APPLICATION

(11) **EP 0 747 067 A2**
(43) Date of publication of application: **11.12.1996**
(21) Application number: 96108504.0
(22) Date of filing: 29.05.1996
(51) Int. Cl.: A61L 27/00, C08L 89/06

(54) **Moldable collagen compositions for hard tissue repair and augmentation**

(30) Priority: 07.06.1995 US 481595
(71) Applicant: COLLAGEN CORPORATION, Palo Alto, California 94303 (US)
(72) Inventor: McMullin, Hugh R., Menlo Park, California 94025 (US); Schroeder, Jacqueline A., Redwood City, CA 94061 (US)
(74) Representative: Schwan, Gerhard, Dipl.-Ing.

(57) **Abstract**

Moldable collagen compositions useful in hard tissue repair and augmentation are disclosed. The compositions comprise nonfibrillar collagen and a particulate material, optionally in combination with biologically active agents. A particularly preferred composition comprises fibrillar collagen, a fiber disassembly agent, and a particulate material. Methods for using the compositions in the repair and augmentation of hard tissue are also disclosed.

## Description

### FIELD OF THE INVENTION

This invention relates generally to compositions particularly useful in hard tissue repair and augmentation; more specifically, it relates to moldable compositions comprising nonfibrillar collagen and a particulate material and, optionally, other components such as growth factors.

### BACKGROUND OF THE INVENTION

A number of bone repair compositions comprising collagen, optionally in combination with particulate materials (particularly ceramics), have been disclosed in the art. For example, U.S. Patent No. 4,440,750, issued April 3, 1984, to Glowacki et al., and commonly owned by the assignee of the present invention, discloses an osteogenic composition comprising a plastic dispersion of particulate demineralized bone and reconstituted collagen fibers dispersed in a continuous aqueous phase having a substantially physiological pH and ionic strength, as well as a method for inducing osteogenesis comprising implanting said composition.

Commonly owned U.S. Patent No. 4,776,890, issued October 11, 1989, to Chu, discloses a method of preparing a mineral collagen matrix comprising dialyzing a mixture of mineral particles and nonfibrillar collagen in acidic solution against a reconstiting medium which comprises a precipitating buffer solution which promotes native collagen fiber formation.

Commonly owned U.S. Patent No. 4,789,663, issued December 6, 1988, to Wallace et al., discloses a method of effecting conductive repair of a bone defect comprising the steps of exposing fresh bone surface comprising living osteoprogenitor cells to the defect and placing into the defect, and into contact with the fresh bone surface, a collagen preparation selected from: (i) a composition consisting essentially of type I collagen derived from demineralized, probe-treated, delipidized bone; (ii) a lyophilized gel of purified atelopeptide reconstituted fibrillar skin collagen; and (iii) mixtures of (i) and purified atelopeptide reconstituted fibrillar skin collagen.

Commonly owned U.S. Patent No. 4,795,467, issued January 3, 1989, to Piez et al., discloses a composition for bone repair comprising a mixture consisting essentially of 60 - 98% by weight of a calcium phosphate mineral component obtained from mineral particles of nonbiological origin in admixture with 2 - 40% by weight of an atelopeptide reconstituted fibrillar collagen. Commonly owned U.S. Patent No. 4,888,366, to Chu et al., issued September 12, 1989, discloses an implantable bone repair composition consisting essentially of 60 - 98% mineral particles, 2 - 40% atelopeptide hypoimmunogenic collagen, and an effective amount of an osteogenic factor (OF) preparation, wherein the implant is a porous rigid solid of compressive modulus greater than 20 N/cm², is homogeneous with respect to its components, and wherein the OF is in biologically active and available form.

Commonly owned U.S. Patent No. 5,264,214, issued November 23, 1993, to Rhee et al., discloses a composition suitable for repair of bone defects comprising collagen chemically conjugated to a synthetic hydrophilic polymer, a suitable particulate material, and a sufficient amount of a fluid pharmaceutically acceptable carrier. Also dislcosed is a composition comprising collagen chemically conjugated to a synthetic hydrophilic polymer, and a suitable particulate material in an amount sufficient to provide a rigid composition.

Commonly owned U.S. Patent No. 5,352,715, issued October 4, 1994, to Wallace et al., discloses an injectable implant composition comprising collagen and biocompatible ceramic particles present in a pharmaceutically acceptable fluid carrier, wherein the ceramic particles have been size selected to have a size distribution in the range from 50 to 250 microns.

U.S. Patent No. 5,083,373 and U.S. Patent No. 5,290,558, issued December 17, 1991, and March 1, 1994, respectively, to McBrayer et al., disclose a flowable bone repair composition comprising a new bone growth-inducing amount of demineralized osteogenic bone powder in a biocompatible carrier selected from a liquid polyhydroxy compound, a liquid polyhydroxy compound derivative, a liquid solution of a solid polyhydroxy compound, and a liquid solution of a solid polyhydroxy compound derivative. Preferably, the carrier is a monosaccharide, disaccharide, oligosaccharide, or a derivative thereof, especially fiuctose, glucose, or a aqueous solution of sucrose or glyceryl monolaurate dissolve in propylene glycol, glycerol, monoacetin, diacetin, and/or liquid polyethylene glycol.

Each publication cited above and herein is incorporated herein by reference in its entirety to describe and disclose the subject matter for which it is cited.

The numerous, previously disclosed bone repair compositions, including those described above, have either been too fluid or too rigid, and have lacked the desired quality of moldability that orthopedic surgeons require in a composition that is to be packed or otherwise implanted into the site of a bone defect.

We now disclose a more moldable bone repair composition comprising collagen, a particulate material, and, optionally, other components, such as growth factors, as well as methods for using these compositions for hard tissue repair and augmentation.

### SUMMARY OF THE INVENTION

In general, previously disclosed collagen-containing bone repair compositions have utilized fibrillar collagen because of its greater mechanical strength and *in vivo* persistence compared to nonfibrillar collagen. Surprisingly, we have found that the use of nonfibrillar collagen in the moldable compositions of the invention has certain advantages in terms of handling and physical properties. In accordance with the present invention, we have discovered that it is possible to use nonfibrillar collagen in combination with a particulate material and, optionally, other components to produce a moldable bone repair composition having a desirable consistency very similar to that of PlayDoh®.

A preferred embodiment of the composition of the invention comprises fibrillar collagen, a fiber disassembly agent, and a particulate material. A particularly preferred embodiment of the composition comprises fibrillar collagen, a biocompatible alcohol, and a particulate material. Another particularly preferred embodiment of the composition comprises fibrillar collagen, a biocompatible alcohol, a particulate material, and a biologically active agent.

Processes for preparing the moldable collagen compositions and methods for using such in the repair and augmentation of hard tissue are also provided. In a general method for repair or augmenting hard tissue, a composition comprising collagen, a fiber disassembly agent, and a particulate material is administered to a hard tissue site in need of repair or augmentation.

### BRIEF DESCRIPTION OF THE DRAWINGS

Figure 1 is a bar graph depicting total fracture repair areas (average for 6 implants) for implant groups containing the following implant materials: (a) no implant material (Control); (b) fibrillar collagen alone; (c) fibrillar collagen + glycerol (1:1); or (d) fibrillar collagen + glycerol + tricalcium phosphate (1:1:2), as evaluated using the rat parietal model. Total fracture repair area refers to the size of the area containing the implant plus any new (hard or soft) tissue ingrowth.

Figure 2 compares the bioactivity of a collagen / glycerol / TGF-β1 formulation with that of a fleshly prepared TGF-β1 standard in acidic ethanol, as measured using the mink lung epithelial cell inhibition assay. The collagen / glycerol mixture with no TGF-β1 was used as a negative control.

Figure 3 compares the bioactivity of a collagen / glycerol / TGF-β1 formulation incubated for 2 months at 4°C with that of a fleshly prepared TGF-β1 standard, as measured using the mink lung epithelial cell inhibition assay.

### DETAILED DESCRIPTION OF THE PREFERRED EMBODIMENTS OF THE INVENTION

In accordance with the present invention, unique, moldable bone repair compositions are prepared by combining nonfibrillar collagen with a particulate material. In order to prepare the moldable collagen compositions of the present invention, it is first necessary to provide collagen and a particulate material.

Collagen is the major protein component of bone, cartilage, skin, and connective tissue in animals. Collagen in its native form is typically a rigid, rod-shaped molecule approximately 300 nanometers (nm) long and 1.5 nm in diameter. It is comprised of three collagen polypeptides which form a tight triple helix. The collagen polypeptides are characterized by a long midsection having the repeating sequence -Gly-X-Y-, where X and Y are often proline or hydroxyproline, bounded at each end by the "telopeptide" regions, which constitute less than about 5 percent (%) of the molecule. The telopeptide region of the collagen chains are typically responsible for the crosslinking between chains and for the immunogenicity of the protein.

In general, collagen from any source may be used to prepare the compositions of the present invention; for example, collagen may be extracted and purified from human or other mammalian source, such as bovine or porcine corium and human placenta, or may be recombinantly or otherwise produced. The preparation of purified, substantially non-antigenic collagen in solution from bovine skin is basically a three-step process involving solubilization, enzyme treatment, and purification, as described in U.S. Patent No. 4,140,537 and U.S. Patent No. 4,488,911, issued February 20, 1979, and December 18, 1984, respectively, to Luck et al., which are incorporated herein by reference. PCT publication No. WO 9403119, filed July 19, 1993, by Palefsky et al., discloses methods of extracting and purifying collagen from the human placenta. PCT Publication No. WO 9416570, filed January 19, 1994, by Berg, discloses methods of producing recombinant human collagen in the milk of transgenic animals, including transgenic cows.

Collagen of any type, including, but not limited to, types I, II, III, IV, or any combination thereof, may be used, although type I is generally preferred. Either atelopeptide or telopeptide-containing collagen may be used; however, when collagen from a xenogeneic source, such as bovine collagen, is used, atelopeptide collagen is generally preferred, because of its reduced immunogenicity compared to telopeptide-containing collagen. The term "collagen" or "collagen material" as used herein refers to all forms of collagen, including those which have been processed or otherwise modified.

Collagen for use in the practice of the invention is preferably noncrosslinked. Noncrosslinked atelopeptide fibrillar collagen is commercially available from Collagen Corporation (Palo Alto, CA) at collagen concentrations of 35 mg/ml and 65 mg/ml under the trademarks Zyderm® I Collagen and Zyderm II Collagen, respectively.

Collagens for use in the present invention are generally in aqueous suspension at a concentration between about 20 mg/ml to about 120 mg/ml; preferably, between about 30 mg/ml to about 90 mg/ml.

Nonfibrillar, rather than fibrillar, collagen is used in the practice of the present invention because it has a more tacky consistency than fibrillar collagen, making it particularly useful in the preparation of compositions intended to be readily moldable. The term "nonfibrillar collagen" as used herein is intended to encompass any modified or unmodified collagen material that is in substantially nonfibrillar form at pH 7, as indicated by optical clarity of an aqueous suspension of the collagen.

Collagens for use in the present invention typically start out in fibrillar form, and then are rendered nonfibrillar by the addition of an appropriate amount of one or more fiber disassembly agent.

Fiber disassembly agents for use in the present invention include, without limitation, various biocompatible alcohols, amino acids, inorganic salts, and carbohydrates. Preferred biocompatible alcohols include glycerol and propylene glycol. Non-biocompatible alcohols, such as ethanol, methanol, and isopropanol, are not preferred for use in the present invention, due to their potentially deleterious effects on the body of the patient receiving them. Preferred amino acids include arginine. Preferred inorganic salts include sodium chloride and potassium chloride. Although carbohydrates, such as various sugars including sucrose, may be used in the practice of the present invention, they are not as preferred as other types of fiber disassembly agents because they can have cytotoxic effects *in vivo*.

When biologically active agents are incorporated into the compositions of the invention, biocompatible alcohols (and, in particular, glycerol) are the preferred fiber disassembly agent, because certain growth factors, such as transforming growth factor beta, have been shown to retain their activity in compositions containing glycerol.

Collagen that is already in nonfibrillar form may also be used to prepare the compositions of the invention. As used herein, the term "nonfibrillar collagen" is intended to encompass collagen types that are nonfibrillar in native form, as well as collagens that have been chemically modified such that they are in nonfibrillar form at or around neutral pH. Collagen types that are nonfibrillar (or microfibrillar) in native form include types IV, VI, and VII. Chemically modified collagens that are in nonfibrillar form at neutral pH include succinylated collagen and methylated collagen, both of which can be prepared according to the methods described in U.S. Patent No. 4,164,559, issued August 14, 1979, to Miyata et al., which is hereby incorporated by reference in its entirety.

Particulate materials for use in the invention include, without limitation, ceramic particles; particulate crosslinked or non-crosslinked fibrillar collagen; poly(lactic) acid (PLA), poly(glycolic) acid (PGA), and copolymers thereof (PLGA); calcium cabonate; calcium sulfate; gelatin beads; polytetrafluoroethylene beads; silicone rubber beads; beads of various hydrogel polymers (such as polyacrylonitrile-polyacrylamide hydrogels); silicon carbide beads; and glass beads. Preferred particulate materials are particulate ceramics and particulate crosslinked fibrillar collagen. As used herein, the term 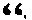 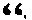 particulate material 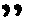 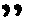 also refers to mixtures containing two or more different types of particulate material, such as those listed above.

The particulate crosslinked fibrillar collagen referenced above is preferably prepared by crosslinking fibrillar collagen with a crosslinking agent selected from the following group: a synthetic hydrophilic polymer (such as a functionally activated polyethylene glycol), a hydrophobic polymer having two or more succinimidyl groups (such as disuccinimidyl suberate or bis(sulfosuccinimidyl) suberate), or a mixture of hydrophilic and hydrophobic polymers, such as those described above. Methods for crosslinking collagen using synthetic hydrophilic polymers such as functionally activated polyethylene glycols are disclosed in U.S. Patent No. 5,162,430 and U.S. Patent No. 5,328,955, issued November 10, 1992, and July 12, 1994, respectively, to Rhee et al.. Methods for crosslinking collagen using hydrophobic polymers and mixtures of hydrophobic and hydrophilic polymers are disclosed in commonly owned, copending EP. application Serial No. 96 102 366.0. Following crosslinking using either a hydrophilic or hydrophobic polymer, or mixtures thereof, the crosslinked fibrillar collagen is dried and then chopped or otherwise mechanically disrupted to form particulates having a size from about 100 microns to about 1000 microns. The tight crosslinked network that can be achieved using hydrophobic polymers such as disuccinimidyl suberate makes hydrophobic polymers ideally suited for the preparation of crosslinked fibrillar collagen particulates for use in the compositions of the present invention.

Preferred ceramics for use in the present invention include any biocompatible particulate ceramic, most preferably, calcium phosphate ceramic. Preferred calcium phosphate ceramics for use in the preparation of compositions of the present invention include tricalcium phosphate particles, hydroxyapatite particles, and mixtures thereof. For best results, preferred ceramics for use in the present invention generally comprise spherical particles having a diameter within the range of about 100 microns to about 1000 microns.

The moldable collagen compositions of the present invention may also comprise one or more biologically active agent. The term 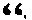 biologically active agent 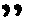 or "active agent" as used herein refers to organic molecules which exert biological effects *in vivo*. Examples of active agents include, without limitation, enzymes, receptor antagonists or agonists, hormones, growth factors, autogenous bone marrow, antibiotics, antimicrobial agents, and antibodies. The term 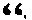 active agent 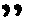 is also intended to encompass various cell types which can be incorporated into the moldable collagen compositions of the invention. The term 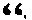 active agent 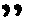 is also intended to encompass combinations or mixtures of two or more active agents, as defined above.

Preferred active agents for use in the present invention include members of the transforming growth factor (TGF) supergene family, which are multifunctional regulatory proteins. Members of the TGF supergene family include the beta transforming growth factors (for example, TGF-β1, TGF-β2, TGF-β3); bone morphogenetic proteins (for example, BMP-1, BMP-2, BMP-3, BMP-4, BMP-5, BMP-6, BMP-7, BMP-8, BMP-9); heparin-binding growth factors (for example, fibroblast growth factor (FGF), epidermal growth factor (EGF), platelet-derived growth factor (PDGF), insulin-like growth factor (IGF)); Inhibins (for example, Inhibin A, Inhibin B); growth differentiating factors (for example, GDF-l); and Activins (for example, Activin A, Activin B, Activin AB). Particularly preferred active agents are the transforming growth factors (TGFs) and bone morphogenetic proteins (BMPs).

Growth factors can be isolated from native or natural sources, such as from mammalian cells, or can be prepared synthetically, such as by recombinant DNA techniques or by various chemical processes. In addition, analogs, fragments, or derivatives of these factors can be used, provided that they exhibit at least some of the biological activity of the native molecule. For example, analogs can be prepared by expression of genes altered by site-specific mutagenesis or other genetic engineering techniques.

Autogenous bone marrow is another preferred biologically active agent which may be incorporated into the compositions of the invention. The bone marrow is generally harvested from the patient at the time of surgery, then mixed with the moldable collagen composition just prior to implantation to a hard tissue site. The bone marrow can be used alone or in combination with other types of biologically active agent, such as growth factors.

Biologically active agents can be added to the composition during preparation or just prior to treatment, as described above for the incorporation of bone marrow. The type and amount of active agent used will depend, among other factors, on the particular site and condition to be treated and the biological activity and pharmacokinetics of the active agent selected.

For biologically active agents other than those which are harvested from the patient just prior to treatment (such as bone marrow, as described above), it is preferred that the agent be incorporated into the composition during preparation so that the agent is released via a sustained-type delivery. In this way, the agent can be released into the tissue site and surrounding areas and exert its intended therapeutic effects over an extended period of time.

In a general method for preparing the moldable collagen compositions of the invention, an aqueous suspension of fibrillar collagen and a fiber disassembly agent are mixed and allowed to incubate for a sufficient period of time to effect disassembly of the collagen fibers (the collagen suspension will be substantially clear at this point). If desired, biologically active agents may be incorporated into the nonfibrillar collagen at this point.

Biologically active agents can either be admixed with the collagen; covalently linked to the collagen using a crosslinking agent such as a functionally activated polyethylene glycol; or affinity bound to the collagen using a binding ligand. Processes for covalently binding biologically active agents such as growth factors to collagen using a synthetic hydrophilic polymer, such as a functionally activated polyethylene glycol, are described in commonly assigned U.S. Patent No. 5,162,430, issued November 10, 1992, to Rhee et al.. Processes for affinity binding biologically active agents to collagen via binding ligand such as heparin are disclosed in commonly owned, copending EP. application Serial No. 96 102 340.5.

The final step in the process for preparing the compositions of the invention comprises mixing the nonfibrillar collagen composition (optionally containing biologically active agents) with a particulate material to form a relatively homogeneous, pliable composition having a doughy texture.

The preferred embodiment of the composition comprises: (i) about 20 to about 60 percent by (wet) weight of an aqueous suspension of fibrillar collagen which comprises about 20 mg/ml to about 120 mg/ml of collagen (preferably, within the range of about 30 mg/ml to about 90 mg/ml of collagen); (ii) about 20 to about 60 percent by weight of a fiber disassembly agent; and (iii) about 20 to about 60 percent by weight of a particulate material, as discussed further in the Examples below. Particularly preferred compositions comprise a fibrillar collagen suspension at a collagen concentration of 65 mg/ml in combination with glycerol and ceramic particles in a weight ratio in the range from 1:1:1 to 1:1:2 collagen suspension : glycerol : ceramic particles (*i.e*., 25 to 33 weight percent collagen suspension; 25 to 33 weight percent glycerol; 33 to 50 weight percent ceramic particles).

Compositions prepared using collagen that is already in nonfibrillar form (*i.e*., compositions not containing fiber disassembly agents) preferably comprise: (i) about 20 to about 80 percent by (wet) weight of an aqueous suspension of nonfibrillar collagen which comprises about 20 mg/ml to about 120 mg/ml of collagen; and (ii) about 20 to about 80 percent by weight of a particulate material.

### USE AND ADMINISTRATION

The moldable collagen compositions of the present invention are particularly suited for use in the repair of hard tissue. As used herein, the term 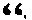 hard tissue 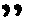 encompasses both bony tissue and cartilaginous tissue.

In a general method for using the compositions of the invention in the repair of hard tissue, the composition, optionally including one or more biologically active agents, is packed or otherwise surgically implanted into the site of a bone or cartilage defect. The composition can be molded *in situ* to the desired shape to fit the defect. Alternatively, the composition can be injected to a hard tissue site through a large bore needle. When compositions containing glycerol are used, the glycerol will eventually diffuse away from the site-of implantation, allowing the collagen to return to its fibrillar form.

The compositions can also he used to augment bone or cartilage, such as in cosmetic surgery applications such as facial reconstruction, or augmentation of various bony or cartilaginous facial features, such as the nose, cheekbones, and chin. The compositions can be applied in a surgical situation to the bone or cartilage site of interest, then sculpted to yield the desired contour.

### EXAMPLES

The following examples are put forth so as to provide those of ordinary skill in the art with a complete disclosure and description of how to make the preferred embodiments of the conjugates, compositions, and devices and are not intended to limit the scope of what the inventors regard as their invention. Efforts have been made to ensure accuracy with respect to numbers used (*e.g*., amounts, temperature, molecular weight, etc.) but some experimental errors and deviation should be accounted for. Unless indicated otherwise, parts are parts by weight, weight ratios are wet weight ratios, molecular weight is weight average molecular weight, temperature is in degrees Centigrade, and pressure is at or near atmospheric.

### Example 1

### Preparation of Collagen / Sucrose / Ceramic Compositions

The following formulations were prepared:

| | Formulation A | Formulation B | Formulation C |
|---|---|---|---|
| Ceramic | 1.5 g | 0.5 g | 1 g |
| Collagen | 3 ml | 2 ml | 1 ml |
| Sucrose | 1 g | 1 g | 1 g |
| Water | 1 ml | --- | --- |

The ceramic component consisted of tricalcium phosphate particles within the size range from about 150 to about 240 microns, obtained from DePuy, Inc. (Warsaw, IN). The collagen was Zyderm® II Collagen without lidocaine (an aqueous suspension of fibrillar collagen at a 65 mg/ml collagen concentration) obtained from Collagen Corporation (Palo Alto, CA). The sucrose was obtained from Sigma (St. Louis, MO).

Formulation A was very sticky in consistency; Formulation B was still sticky, but more moldable than Formulation B; Formulation C had a consistency very similar to that of PlayDoh®.

The following formulations were prepared, using the same components described above, to determine the effect of varying the amount of sucrose:

| | Formulation D | Formulation E | Formulation F |
|---|---|---|---|
| Ceramic | 1.5 g | 1.5 g | 2 g |
| Collagen | 1 ml | 1 ml | 1 ml |
| Sucrose | 0.5 g | 1 g | 1.5 g |

Formulation D was translucent (rather than transparent), indicating incomplete fiber disassembly of the collagen. This formulation also tended to fall apart easily. Formulation E was transparent and had excellent, 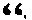 PlayDoh-like 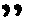 handling characteristics. Formulation F was also transparent and had excellent handling characteristics, but was not quite as elastic as formulation E.

The following additional formulations were prepared using the same components described above:

| | Formulation G | Formulation H | Formulation I |
|---|---|---|---|
| Ceramic | 1.5 g | 1.1 g | 1.1 g |
| Collagen | 1 ml | 1 ml | 1 ml |
| Sucrose | 2 g | 1 g | 0.5 g |

Formulations G, H, and I were allowed to dry overnight at room temperature. Each of the dried formulations was placed in a phosphate-buffered saline (PBS) solution. Formulation H held its shape after 3 hours in PBS, indicating Bat the formulation would remain cohesive *in vivo* when in contact with bodily fluids. The other two formulations fell apart.

The results of these experiments indicate that the formulations having the most desirable handling characteristics contained approximately 1 ml of fibrillar collagen suspension (at a collagen concentration of 65 mg/ml) in combination with 1 - 2 g of tricalcium phosphate particles and 1 - 1.5 g of sucrose.

### Example 2

### Preparation of Collagen / Glycerol / Ceramic Compositions

The following formulations were prepared:

| | Formulation J | Formulation K | Formulation L |
|---|---|---|---|
| Ceramic | 1.1 g | 1.1 g | 1.1 g |
| Collagen | 1 ml | 1 ml | 1 ml |
| Glycerol | 2 ml | 1 ml | 0.5 ml |

The ceramic component consisted of tricalcium phosphate particles within the size range from about 150 to about 240 microns, obtained from DePuy Inc. (Warsaw, IN). The collagen was Zyderm® II Collagen without lidocaine (an aqueous suspension of fibrillar collagen at a 65 mg/ml collagen concentration) obtained from Collagen Corporation (Palo Alto, CA). The glycerol was obtained from Sigma (St. Louis, MO).

Formulation K was found to be the best of the three formulations in terms of moldability and general handling characteristics. Formulation J was found to be too sticky and Formulation L fell apart.

### Example 3 In vivo Evaluation of Collagen / Glycerol / Ceramic Compositions

An *in vivo* study was performed to evaluate bone ingrowth into the collagen / glycerol / ceramic compositions of the invention. The rat parietal bone fracture repair model was used, with twelve male Sprague-Dawley rats receiving bilateral fractures. Each rat received two 0.05-ml implants (one in each fracture site) selected from the four materials listed below, for a total of 6 evaluation sites per material:
- Zyderm® II Collagen (65 mg/ml collagen concentration);
- Zyderm II Collagen + glycerol;
- Zyderm II Collagen + glycerol + tricalcium phosphate;
- No implant material (Control).

The glycerol-containing formulations were prepared by mixing the contents of a 5-cc syringe containing 5 grams of Zyderm II Collagen with the contents of a 5-cc syringe containing 5 grams of autoclaved (sterilized) glycerol. The collagen and glycerol were mixed by syringe-to-syringe mixing using a three-way stopcock and employing 100 passes of material between the two syringes. The collagen / glycerol mixture was stored at 4°C.

The ceramic-containing formulation was prepared by mixing the collagen / glycerol mixture with an equivalent quantity of tricalcium phosphate at the time of implantation to achieve a final formulation containing 25% collagen suspension (at a collagen concentration of 65 mg/ml), 25% glycerol, and 50% tricalcium phosphate (TCP).

The animals were sacrificed and the implants excised and examined histologically at 28 days post-implantation. No adverse response was observed with any of the implant materials: the brain and tissue surrounding the implant material appeared normal in all cases. There was no significant difference between implants containing collagen alone and implants containing collagen + glycerol. Implants containing collagen, with or without glycerol, contained abundant amounts of osseous tissue of mostly primary structure (trabecullar).

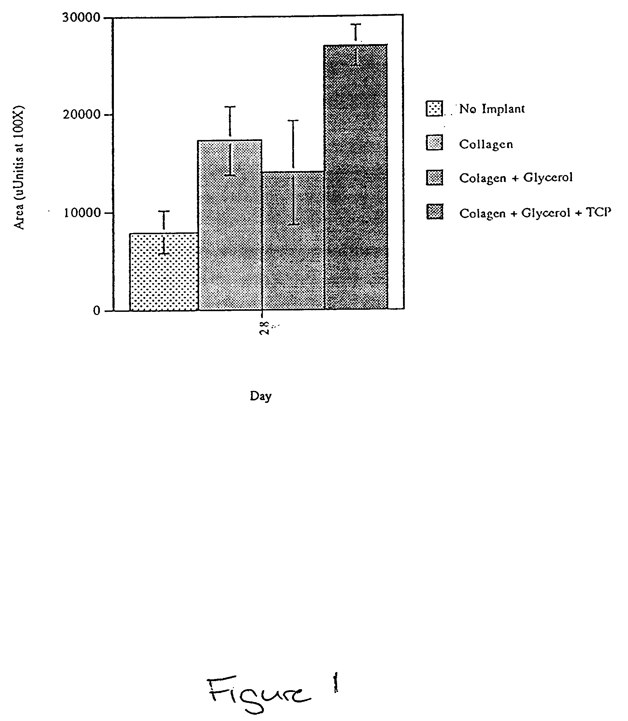

A bar graph depicting total fracture repair areas (average for 6 implants) for each of the four implant groups (including the control group containing no implant material) is presented in Figure 1. Total fracture repair areas were measured using a micrometer at 100x magnification at 20 unit intervals. Total fracture repair area refers to the size of the area containing the implant plus any new (hard or soft) tissue ingrowth. The original fracture areas and implant areas were substantially the same for all implants at the beginning of the study. Therefore, a relatively larger fracture repair area indicates one or both of two things: (a) there has been more tissue ingrowth into the fracture area, indicating that the implant material is more conducive to tissue ingrowth than the other formulations; (b) the implant has maintained its size better than other implants, indicating greater *in vivo* persistence of the implant material.

As shown in Figure 1, the largest fracture repair areas were seen for implants containing the collagen / glycerol / TCP formulation.

### Example 4

### Preparation of Collagen / Glycerol / Ceramic Compositions With Growth Factors

Zyderm® II Collagen and glycerol were mixed together in a 1:1 ratio, as described in Example 3, above. Forty (40) micrograms of TGF-β1 (obtained from Genentech, Inc., South San Francisco, CA) was added to the collagen / glycerol mixture. Finally, tricalcium phosphate was added to the collagen / glycerol / TGF-β1 mixture in a 1:1 weight ratio of TCP to collagen / glycerol / TGF-β1 mixture (for a final weight ratio of 1:1:2 collagen suspension: glycerol : TCP).

### Example 5

### In vitro Activity of Collagen / Glycerol Compositions Containing Growth Factors

The activity of a formulation containing 40 µg/ml TGF-β1 in collagen / glycerol (in a 1:1 weight ratio) after 2 month storage at 4°C was measured by its ability to inhibit mink lung epithelial cell (ATCC-MvILu CCL-64) proliferation, according to the method described by Tada et al. (J. Immunol., 1991, 146:1077-182). Cellular inhibitory response was measured using a chromogenic substrate for acid phosphatase. A quantitative estimate of TGF-β1 activity was determined by the inhibition of proliferation of mink lung epithelial cells by comparison to a standard curve of TGF-β1. Formulations were tested for activity by diluting the formulation directly in the tissue culture media.

The collagen / glycerol / TGF-β1 formulation was compared with a freshly prepared TGF-β1 standard in acidic ethanol as the positive control. The collagen / glycerol mixture with no TGF-β1 was used as a negative control.

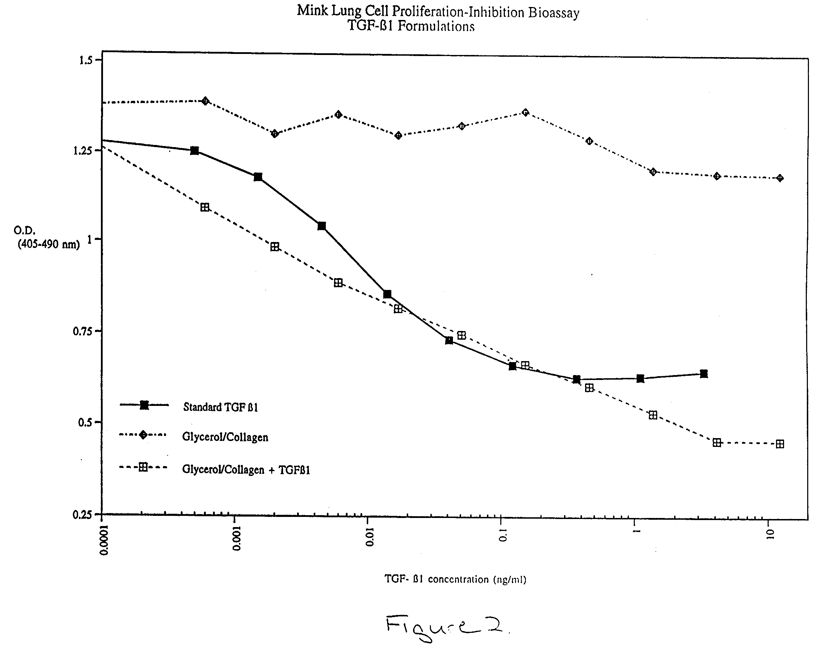

As shown in Figure 2, the collagen / glycerol / TGF-β1 formulation appeared to be roughly equivalent to the TGF-β1 standard at inhibiting mink lung epithelial cell proliferation at all concentrations tested, as evidenced by low absorbance values in the acid phosphatase assay.

### Example 6

### In vitro Stability of Collagen / Glycerol Compositions Containing Growth Factors

The stability of a formulation containing 40 µg/ml TGF-β1 in collagen / glycerol (in a 1:1 weight ratio) after 2 month storage at 4°C was measured using the mink lung epithelial cell inhibition assay described in Example 5, above. A freshly prepared TGF-β1 standard in acidic ethanol was used as the positive control.

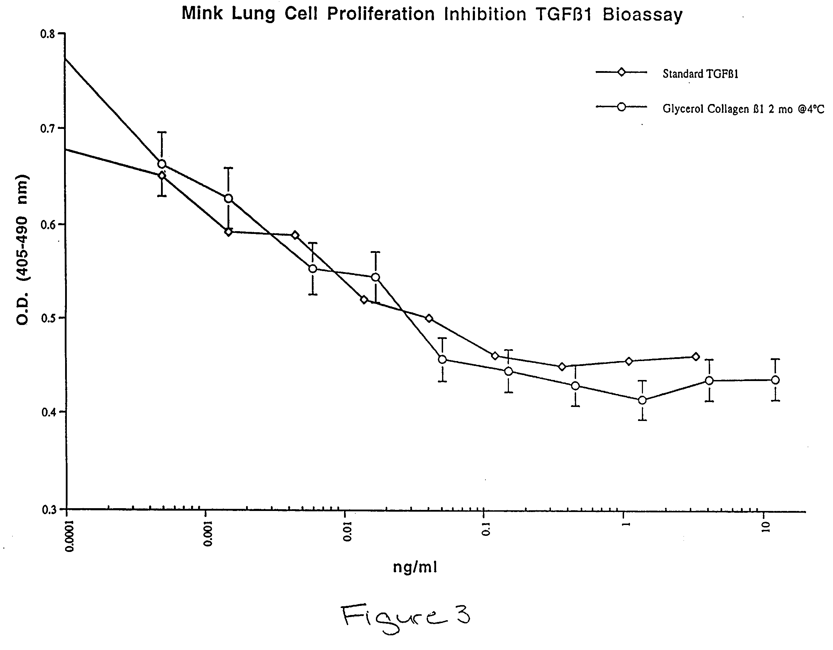

As shown in Figure 3, the collagen / glycerol formulation containing TGF-β1 was stable and successfully inhibited mink lung epithelial cell proliferation after 2 month storage at 4°C.

## Claims

1. A moldable composition suitable for use in the repair and augmentation of hard tissue, which composition comprises nonfibrillar collagen and a particulate material.

2. The composition of claim 1, wherein the nonfibrillar collagen is prepared by mixing fibrillar collagen with a fiber disassembly agent.

3. The composition of claim 1 or 2, wherein the nonfibrillar collagen is selected from the group consisting of: type IV collagen, type VI collagen, and type VII collagen.

4. The composition of claim 1 or 2, wherein the nonfibrillar collagen is a chemically modified collagen selected from the group consisting of methylated collagen and succinylated collagen.

5. A moldable composition suitable for use in the repair and augmentation of hard tissue, which composition comprises fibrillar collagen, a fiber disassembly agent, and a particulate material.

6. The composition of claim 2 or 5, wherein the fiber disassembly agent is selected from the group consisting of: a biocompatible alcohol, an amino acid, an inorganic salt, and a carbohydrate.

7. The composition of claim 4, wherein the fiber disassembly agent is a biocompatible alcohol selected from the group consisting of glycerol and propylene glycol.

8. A moldable composition suitable for use in the repair and augmentation of hard tissue, which composition comprises fibrillar collagen, a biocompatible alcohol, and a particulate material.

9. A moldable composition suitable for use in the repair and augmenttion of hard tissue, which composition comprises fibrillar collagen, a biocompatible alcohol, a particulate material, and a biologically active agent.

10. The composition of claim 8 or 9, wherein the biocompatible alcohol is selected from the group consisting of glycerol and proplylene glycol.

11. The composition of any one of the preceding claims, wherein the particulate material is selected from the group consisting of: ceramic particles; particulate crosslinked or non-crosslinked fibrillar collagen; poly(lactic) acid (PLA), poly(glycolic) acid (PGA), and copolymers thereof (PLGA); calcium carbonate; calcium sulfate; gelatin beads; polytetrafluoroehtylene beads; silicone rubber beads; beads of various hydrogel polymers (such as polyacrylonitrile-polyacrylamide hydrogels); silicon carbide beads; and glass beads.

12. The composition of claim 11, wherein the particulate material comprises ceramic particles.

13. The composition of claim 12, wherein the ceramic particles are calcium phosphate ceramic particles selected from the group consisting of: tricalcium phosphate particles, hydroxyapatite particles, and mixtures thereof.

14. The composition of claim 11, wherein the particulate material comprises particulate crosslinked fibrillar collagen.

15. The composition of claim 14, wherein the particulate crosslinked fibrillar collagen is crosslinked using a crosslinking agent selected from the group consisting of: a synthetic hydrophilic polymer, a hydrophobic polymer having two or more succinimidyl groups, and mixtures htereof.

16. The composition of any one of the preceding claims further comprising an effective amount of one or more biologically active agent.

17. The composition of claim 16, wherein the biologically active agent is a growth factor selected from the group consisting of: enzymes, receptor antagonists or agonists, hormones, growth factors, autogenous bone marrow, antibiotics, antimicrobial agents, and antibodies.

18. The composition of claim 17, wherein the biologically active agent is a growth factor, wherein the growth factor is a member of the transforming growth factor supergene family.

19. The composition of claim 18, wherein the growth factor is selected from a transforming growth factor and a bone morphogenetic protein.
